(19) Europäisches Patentamt
European Patent Office
Office européen des brevets

(11) Publication number: **0 249 416**
**A2**

(12) # EUROPEAN PATENT APPLICATION

(21) Application number: **87305012.4**

(22) Date of filing: **05.06.87**

(51) Int. Cl.[4]: **C08G 81/00** , C08G 77/42 , A61L 27/00

(30) Priority: **06.06.86 US 872197**

(43) Date of publication of application:
**16.12.87 Bulletin 87/51**

(84) Designated Contracting States:
**AT BE CH DE ES FR GB GR IT LI LU NL SE**

(71) Applicant: **THORATEC LABORATORIES CORPORATION**
**2448 Sixth Street**
**Berkeley, CA 94710(US)**

(72) Inventor: **Ward, Robert S.**
**323 Lowell Lane East**
**Lafayette California 94549(US)**

(74) Representative: **Allard, Susan Joyce et al**
**BOULT, WADE & TENNANT 27 Furnival Street**
**London EC4A 1PQ(GB)**

(54) **Method for modifying polymer surfaces with surface-active polymer blocks tethered to non-surface-active components.**

⑤ A method is provided for modifying the surfaces of polymer substrates by including therein additives. The additives are characterized by a surface-active block polymer chain covalently bonded to a component which imparts desirable surface characteristics to the substrate.

EP 0 249 416 A2

Xerox Copy Centre

# METHOD FOR MODIFYING POLYMER SURFACES WITH SURFACE-ACTIVE POLYMER BLOCKS TETHERED TO NON-SURFACE-ACTIVE COMPONENTS

The present invention provides a method for modifying polymer surfaces using novel block polymers comprising surface-active blocks covalently bonded to non-surface-active components. In particular, the present invention provides novel surface-modifying polysiloxane-poly(oxazoline) block copolymers and novel oligomer intermediates useful for the preparation thereof.

While natural and synthetic polymers have many uses in that they are inexpensive and readily formable by molding, extrusion, casting, and the like, into virtually any desired shape to form an article having mechanical strength, in many instances satisfactory performance of the polymer, hereinafter also referred to as the base polymer, often requires the use of one or more functional additives. Additives are generally used in concentrations of from less than one tenth percent to ten percent or more by weight to enhance a wide range of properties related to the stability and utility of the configured polymer article.

In many cases the additive is used to alter and/or improve a property which involves the surface of the configured article. Surface modification is required, for instance, in the case of stabilizing additives used to inhibit oxidation, since the initial region of attack from atmospheric oxygen is at the interface between the atmosphere and the polymer surface. Similarly, dissipation of static charge occurs at the surface of the configured polymer article such as a fiber or film. However, many polymer additives have no tendency to concentrate at the polymer's surface where their concentration should be the highest. Thus, in order to get sufficient surface concentration of such a stabilizer or property modifier, it is often necessary to employ high bulk concentrations of the additive in the polymer. This is expensive and can lead to degradation of some of the desirable physical/mechanical properties of the base polymer. In some cases, such as antistatic additives for critical applications, it is necessary to apply a topical coating rather than an internal additive, in order to get the desired level of performance. In general, topical coatings are temporary and readily removed from the polymer surface during use. Furthermore, coatings involve an additional post-fabrication manufacturing step which further detracts from the desirability of this approach.

It would thus be desirable to devise a means of imparting surface activity to a polymer additive or property modifier in a base polymer, while minimizing the bulk concentration needed to obtain satisfactory performance and also improving the permanence of the additive in the base polymer.

Surface activity is the tendency for a material originally dispersed uniformly throughout a bulk phase to accumulate at a surface or interface at a concentration significantly exceeding its bulk concentration. Surface activity is well known in low viscosity liquid systems. Surfactants (e.g. detergents), having so-called amphipathic structure, will accumulate at the surface of a liquid and lower that liquid's surface tension. The enrichment of surfactant at the interface is driven by the minimization of surface free energy of the system, which occurs when the surfactant populates the surface at the expense of the higher surface energy solvent (e.g . water).

In our British Patent 2073219B, terpolymers and multipolymers as surface-modifying additives are disclosed for a variety of base polymers, in particular, those used in contact with blood in a medical device. In such terpolymers and multipolymers, a silicone block is often present along with a short, rigid block or segment, having a high glass transition temperature or crystalline melting point. In addition, a polar or hydrophilic block may be present which has a low glass transition temperature relative to the short hard block. The surface energy of the polar or hydrophilic block is generally higher than that of the base polymer to be modified. When contacted with a low energy environment like air, these multipolymers orient to give surfaces rich in the low energy block of the multipolymer. In more polar or high energy environments (e. g. water or blood) the same multipolymers reorient to give surfaces rich in the high energy block of the additive. In this way these terpolymers and multipolymers help the surface minimize its interfacial energy no matter what the nature of the environment in which it is immersed or contracted. The polar blocks of these terpolymers thus appear to give a pronounced amphipathic (hydrophobic/hydrophilic) character to the surface-modifying additive. The more pronounced the difference in polarity between the low energy and high energy blocks, the more effective the additives are likely to be at minimizing interfacial energy in any environment. However, except for providing hydrophilicity (and high surface energy) the British patent does not teach that the polar blocks of the multipolymers provide any other function.

It is thus an object of the present invention to prepare copolymers, terpolymers and multipolymer additives which contain functional blocks or pendent groups in addition to a surface-active block. By functional blocks or groups it is meant any molecular or elemental species which provides, through chemical or physical processes, a desired modification to the base polymer and which, molecular or elemental species as a pure material, does not possess sufficient surface activity in the base polymer for optimum performance.

It is a further object of the present invention to provide novel surface modifying additives comprising surface-active polymer blocks "tethered" to non-surface-active components.

The present invention provides a method for modifying base polymers with block polymer additives. The block polymer additives are prepared to impart a predetermined surface property to the base polymer and the additive comprises a surface-active component which is surface-active in the base polymer comprising a block polymer chain of molecular weight generally greater than about 400; and a functional component characterized by one or more predetermined surface properties, linked through covalent bonds to the surface-active component, wherein the functional component is essentially non-surface-active in the base polymer and wherein the linkage between the functional component and the surface-active component is of sufficient length to maintain the functional component sufficiently close to the surface-active component in the base polymer to impart the predetermined surface property to the base polymer. The block polymer additive is also substantially nonexudable from the base polymer, and is thermoplastic or substantially soluble in organic solvents, as well as substantially insoluble in aqueous solvents in most cases. Novel classes of such block polymer additives and methods for modifying base polymers with such additives are also provided.

According to the present invention the surfaces of base polymers may be modified by use of an additive where the additive comprises a block polymer chain or molecular weight greater than 400 in at least most cases and which is ordinarily surface-active in most polymers, such as a silicone block, a fluorocarbon block or a hydrocarbon block, which in turn is covalently bonded to a functional component, the properties of which are desired at the surface of the base polymer. By using such additives the functional component whose presence is desired at or near the surface of the base polymer tends to concentrate at the surface because it is covalently bonded, or as referred to herein, "tethered" to the surface-active component.

Some examples of functional blocks or groups are those which can impart to the polymer to which they are added, improvements in any of the following properties: oxidation resistance, radiation resistance, chemical resistance, hydrolytic stability, thermal stability, coefficient of friction, drag (reduction), etchability or etch resistance by plasmas, stain repellency, launderability, release properties, reduced blocking, wettability, anticoagulant activity, antimicrobial activity, (reduced)mineralization (e.g. in vivo calcification), static charge dissipation, (reduced) overgrowth by marine organisms, surface chemical reactivity, selective adsorption/absorption, selective affinity, reduced toxicity, increased compatibility with biological species, increased spreading and/or viability of cell cultures, increased adhesion and bond strength, control of refractive index, color intensity, dyeability, gloss, water absorption, barrier properties, permeability, tear strength, crack propagation, tensile properties, flammability, explosive characteristics, tactile properties and "hand" rheology, film forming ability, control of tack, indicator properties, photosensitivity, magnetic receptivity, electrical conductivity, dielectric strength and any other property that can be created or affected by tethering a functional moiety to a surface-active polymer to render the functional moiety surface active in the base polymer(s) to which it will be added.

The additives according to the present invention have three primary characteristics: when they are present in the base polymer they are surface-active, the functionality of the functional component imparts a desired surface characteristic to the base polymer and, the additive remains substantially permanently in the base polymer.

Regarding the feature of the functionality of the functional component it is recognized that the functional component or pendent group must be selected from those chemical structures which will be sufficiently preserved so that the functional block or pendent group retains its essential properties once it is incorporated into the additive and base polymer. Therefore the functional component must be substantially physically and chemically stable throughout the processing utilized to prepare the additive and to incorporate the additive into the base polymer so as not to destroy the ability of the functional block or pendent group to stabilize or enhance the surface properties of the base polymer.

As for the feature of surface activity of the additive, the block length of the surface-active component must be sufficiently long, (i.e. its molecular weight must be sufficiently high) to provide the necessary surface activity to the polymer additive. As an example, in the case of polydimethylsiloxane surface-active blocks, the necessary molecular weight corresponds approximately to a block length in which a glass

transition temperature of around -110°C or less may be detected by differential scanning calorimetry. However, in most additives according to the present invention this would correspond to a DP of at least 8 or a molecular weight greater than 400. Again, the chemical structure of the surface-active component must be preserved to be substantially stable chemically and physically through the processing required to prepare the additive and to incorporate it into the base polymer. Additionally, the additive comprising the surface-active component and functional component should desirably be thermoplastic and/or soluble (prior to its incorporation into the base polymer) in order to assure optimal dispersion within the base polymer during processing and to provide sufficient mobility for surface activity once incorporated into the base polymer. The additives according to the present invention are also desirably relatively insoluble in water since, for most useful applications, the base polymer will be in a physical admixture with the additive and since in most applications the surface of the base polymer would be subjected to aqueous environments, the water insolubility is a desirable trait in order to avoid leaching of the additive from the base polymer.

An important feature of the additives according to the present invention are the relative permanence once incorporated into the base polymer. Thus, more than one functional block may be provided in a single additive in order to obtain more than one type of stabilization or property enhancement. By including molecular blocks or segments having a high cohesive energy density characterized by a major thermal transition above room temperature, -20°C, such molecular blocks or segments may be capable of forming glassy or crystalline domains within the additive, thereby converting the entire additive molecule into a solid. Such high cohesive energy density blocks are preferably characterized by a DP of less than about 10 or a molecular weight of less than about 10,000 but may be larger depending the overall DP of the additive polymer.

Conversion of the additive to a solid will generally enhance its permanence within the base polymer. Such solids are still capable of migration to the surface of the configured polymer article, but are constrained from leaving the surface by chain entanglement and/or specific interactions with the base polymer and the high vapor pressure and low mobility associated with the solid phase.

However, depending upon the nature of the functional component, its structure, and the final molecular weight of the additive, the additive may possess sufficient permanence and surface activity in the base polymer without the presence of additional high cohesive energy density blocks or segments. Thus, if the additive, under normal or use temperature is a solid, this would indicate that hard blocks would not be required. However, if the pure additive polymer exists in the liquid state in the absence of hard blocks, then this would indicate that hard blocks should be included within the additive. In such a case the hard blocks would be needed to prevent exudation and eventual loss of the additive from the base polymer.

In another embodiment of the invention, the permanence required of the additive within the base polymer is achieved by permanent chemical (covalent or otherwise) attachment to the base polymer. Thus if both the base polymer and the additive polymer contain thermolabile chemical groups, interchange reactions will effectively form covalent bonds between the additive and base polymer when the two are exposed, for example, to elevated temperatures.

Thus, if the base polymer is a polyester and the additive polymer contains a polyester block, when a simple mixture of the two are processed in the melt, ester groups on both polymers may dissociate into acids and alcohols. Upon reforming in the melt or after processing, new ester groups will be formed between the base polymer and the polymeric additive. The result is a different composition having physical/mechanical properties dominated by the base polymer and the property and/or stability enhancements provided by the polymeric additive portion.

In addition to polyester, several other classes of polymers have thermolabile bonding groups that may be employed to achieve chemical bonding between base polymer and additive polymer. These include polyamides, polyesters, polycarbonates, polyurethanes, polyureas among others. Combinations of these thermolabile groups can also be employed. One type of thermolabile group can be present in the base polymer while another may be present in the additive polymer. For example, a polyamide base polymer and a polyester-containing additive polymer can undergo interchange reactions, since dissociation of the amide yields an acid and an amine, while dissociation of the ester yields an acid and an alcohol. The alcohol attached to the additive polymer fragment can then react with the acid from the original amide group to produce a new ester group connecting base polymer and additive polymer. The remaining acid group attached to the additive polymer fragment can react with the amine on the base polymer to produce a new amide group connecting the additive and base polymers. Repetition of this process can lead to structures that are difficult to define accurately, but which clearly contain covalent bonds between additive polymer and base polymer and therefore differ from simple blends.

In those systems formed from the above interchange reactions the resulting multipolymer will be characterized by molecular chains which are surface-active owing to the presence of the silicone (or other surface-active) blocks originally part of the additive polymer. However, a higher original bulk concentration of the polymeric additive may be required in some cases. This is balanced by the improved permanence of the additive in the resulting composition, which is not merely a physical mixture of base polymer and additive, but a different composition-of-matter involving formation of new chemical bonds.

In those additives which may undergo interchange reactions with the base polymer, it is necessary to provide chemical groups between the functional and surface-active blocks of the additive polymer which are not thermolabile or are significantly less thermolabile at the processing or reaction temperature than the thermolabile groups in the base polymer. In order to preserve the desirable combination of surface-active blocks in close proximity to the functional blocks, it is necessary to preserve the connecting groups which bind the two blocks together. This is true even when the two types of blocks are interconnected by (high cohesive energy density) hard blocks or segments. However, the requirement of thermally-stable connecting groups between surface-active and functional groups does not apply when the base polymer/additive polymer mixture is not intended to be exposed to high temperatures. The use of the surface-active/functional polymeric polymer additive of this invention in solvent-cast paints, coatings and films is one case where thermally-stable connecting linkages may not be required.

The polymeric additive of this invention can be prepared in the form of block, graft or segmented copolymers, according to general methods which are well known in the art (Block Copolymers Overview and Critical Survey by J. E. McGrath and A Noshay, Academic Press, l977). In order to achieve covalent bonding to (in particular) the functional blocks, it is necessary that they possess appropriate reactivity towards the surface-active and the hard blocks or segments or their successors.

Although not wanting to be limited to this case, it may often be desirable to preform the functional blocks or segments, providing one or more reactive groups which can be used in subsequent reaction steps to bond the functional blocks to the surface-active blocks or the hard segments. Alternatively, it is possible to prepare the functional group in situ during the synthesis of the additive polymer. The former method is preferred when attempting to incorporate an existing functional group into the surface-active polymeric additive. The incorporation of one or more hindered phenolic antioxidant blocks or pendant groups into a polymer additive containing polydimethylsiloxane and polycaprolactone blocks could be accomplished, for instance, through reactions between chemical groups on the hindered phenolic structure and the caprolactone and/or siloxane blocks.

Like the functional blocks or groups, the surface-active blocks can be prepared as preformed oligomers which are then coupled to the functional groups in the final synthesis reaction. Alternatively, the surface-active blocks can also be prepared in situ (e.g. by the ring-opening polymerization of cyclic siloxanes initiated at a site on the functional block) by general methods known in the art (McGrath and Noshay). Similar considerations exist for the hard block if one or more are to be included in the additive polymer.

The overall structure of the additive of the present invention can vary broadly within the above specification in that they can be prepared in a variety of block, graft and segmented copolymer configurations. If S is a surface-active block such as polydimethysiloxane, $F_1$ is a functional block providing "function l," $F_2$ is a functional block providing "function 2" and H is a high cohesive energy density block on hard block, exemplary structures for additives of the present invention are

Block and Segmented Structure:

l .$F_1$-S
2. $[F_1\text{-}S]_n$
3. $F_1$-S-$F_2$
4. $[F_1\text{-}S\text{-}F_2]_n$
5. $F_1$-$F_2$-S
6. $[F_1\text{-}F_2\text{-}S]_n$
7. $F_1$-H-S
8. $[F_1\text{-}H\text{-}S]_n$
9. $[S\text{-}H\text{-}S\text{-}F_1]_n$
l0. S-$F_1$-S
ll. $[S\text{-}F_1\text{-}S]_n$
l2. $[S\text{-}F_1\text{-}S\text{-}H]_n$

Graft Structure:

1. $$[\underset{\underset{F_1}{|}}{(S)}_a - H_x - \underset{\underset{F_2}{|}}{(S)}_b - H_y - (S)_c]_n$$

2. $$[\underset{\underset{(S)_x}{|}}{F_1} - (F_1)_a - \underset{\underset{(S)_y}{|}}{F_2} - (F_2)_b]_n$$

A preferred embodiment of the present invention is the application to additives which will impart calcification resistance to the base polymer. Elastomers used in the fabrication of biomedical devices and prostheses which are used in prolonged contact with blood are prone to accumulate calcium deposits from the blood. Prosthetic heart valves, synthetic vascular grafts, artificial hearts, cardiac assist devices and other chronically implanted blood or tissue-contacting devices, particularly those which are continually flexed (e.g. in synchrony with the heart), can become disfunctional and/or thrombogenic as a result of the "calcification process" which rigidizes the polymer and roughens its surface. Calcification of biomaterial implants is particularly pronounced in children, whose level or circulating blood calcium (needed for bone growth) is higher than in adults. At least two approaches may be used to discourage calcification of biomaterials. One approach prevents the nucleation of crystals of calcium salts on or within the base material. The other approach discourages the growth of crystals once nucleation has occurred.

Materials such as sodium dodecylsulphate (SDS) are known as nucleation preventers in biological tissue, but without bonding the water-soluble SDS to the tissue. In fabricating a prosthetic heart valve with a smooth blood-contacting surface from a synthetic elastomer, it is unlikely that such an approach would be effective. The nonporous nature of the elastomer may prevent uptake of the SDS solution. If incorporated into the polymer itself, the water solubility of the SDS would result in its removal by leaching when the device was exposed to blood. Removal of material by leaching would result in porosity in the blood contacting surface which could further promote blood clots, calcification and a reduction in strength of the polymer. Another example of a method aimed at inhibiting crystal growth, is the controlled release of certain diphosphonate-containing drugs from a polymer matrix, with no attempt to bond the diphosphonate to the matrix or base polymer. The problems associated with leaching of SDS would also be encountered with controlled release of diphosphonates.

According to the present invention the permanence of the anticalcification additive (functional component) in the polymeric biomaterial is obtained in order to maximise the useful lifetime of the implantable device. Since the calcification is believed to be initiated (in flaws) at the surface of the biomaterial, it is also an advantage of the present invention to concentrate the anticalcification agent at the blood or tissue-contacting surface. Furthermore, according to the present invention the anticalcification agent may be rendered nonsoluble and nonexudable in order to keep it permanently present in the surface.

The choice of the particular surface active component to be utilized will depend upon several factors, including the nature of the base polymer. Block copolymer chains which are known to be surface-active in various types of base polymers are known, for example, polydialkylsiloxanes are generally surface-active in a variety of base polymers. In such an instance, the polydialkylsiloxanes may be chemically linked to the functional component, depending upon the functional groups available on the polydialkylsiloxane and on the functional component which are available for such linkage. Such linkages may be made by those of ordinary skill in the art using conventional techniques. The chemical linkage between the surface-active blocks should be of a length sufficient so that the functional component will be held close to the surface-active component, and therefore, also close to the surface of the base polymer. While not intending to be bound by any particular theory, it is believed that in most instances the surface-active component will be within one or two molecular monolayers of the surface of the base polymer, thereby also holding the functional component close to the surface of the base polymer. If the chemical linkage between the surface-active component and the functional component is too long, then it would reduce the efficacy of the functional component since it would be possible for the surface-active component to be held within one or two monolayers of the surface of the base polymer, with the functional group being retained sufficiently deep under the surface of the base polymer so as to substantially diminish or even eliminate the efficacy of the functional group as a surface characteristic of the base polymer.

An exemplary surface-active component is a silicone of the formula

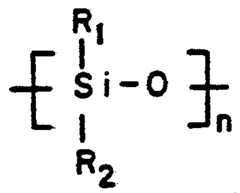

wherein $R_1$ and $R_2$ are alkyl groups of l to 4 carbon atoms, fluoroalkyl of l to 4 carbon atoms or aryl of 6 to l0 carbon atoms; and n is from l to about 200.

The surface-active component will be usually a polymeric block. Block polymers may be linear, cyclic, graft or branched structures. The preferred block polymer components are linear or graft.

The non-surface-active component of the additives according to the present invention may be any component which would impart desirable surface properties to the base polymer composition. The invention is particularly useful in providing additives wherein the non-surface-active block is hydrophilic (i.e. polar) and is characterized by a critical surface tension or liquid surface tension greater than the critical surface tension or liquid surface tension, respectively, of the base polymer. The non-surface-active component may also be a block polymer, such as polyethylene-oxide and polyoxazoline. The non-surface-active component imparts desired surface properties to the base polymer composition, therefore the non-surface-active component may be an anticoagulant, polymer stabilizer, antimicrobial agent, an antistatic agent, an antioxidant, an ultraviolet light stabilizer, a lubricant or a pharmaceutical agent, for example.

The surface-active component and non-surface-active components in the additive according to the present invention will be covalently linked, i.e., the non-surface-active agent will be tethered to the surface-active agent. Preferably, the surface-active and non-surface-active components will be linked by thermally and hydrolytically-stable chemical bonds.

The additives according to the present invention may be prepared by first preparing an oligomer of the surface-active component. The following discussion will, for convenience, refer to the surface-active component as a polysiloxane, since that is a preferred component, however the methods are intended to be generalized for any surface-active component. As used hereinbelow, the term oligomer will refer to a polymer, which may be a block copolymer as well, containing at least 2 and up to about 200 recurring monomer units. The polysiloxane oligomer may be synthesized to contain either terminal or pendant reactive groups or both. These reactive groups may then be coupled to the non-surface-active component to form the tethered additive.

An alternative method is to prepare a non-surface-active component having at least one reactive functionality so that the reactive functionality may be utilized to initiate the formation of a polysiloxane block. In general, methods are known in the art for making block copolymers, multipolymers, or graft copolymers whereby functionalized species may be used to attach to a polysiloxane block, hydrocarbon block or fluorocarbon block.

The length of the polysiloxane block and, if present in block form, also the non-surface-active component, should be such that the blocks are long enough to impart the desired surface characteristics, but not so long as to substantially impede the surface concentration of the blocks. Therefore, the blocks should contain less than about 200 recurring monomer units.

The preferred polysiloxane block additives of the invention are compatible and miscible with a wide variety of base polymers. As used herein, the term compatibility means the ability of the copolymer to remain homogeneously dispersed within the bulk of the base polymer at a concentration of about 0.l% or more by weight of the additive. These base polymers include polymers such as polyacrylonitrile, polyvinylchloride, polyvinylacetate, polyvinylidene chloride, poly(methylmethacrylate), polystyrene, polyphenylene oxide, poly(ethylacrylate), polyacrylic acid, polypropylene, polyethylene, polyethylenepropylene copolymers, polyethylene-vinyl acetate copolymers, polyurethanes, polycarbonates, polyamides, polysulfones, polyesters, polyimides, polyetherpolyesters, poly(acrylonitrile-butadiene-styrene), polybutadiene, polyisoprene, styrene-butadiene-styrene block copolymers, styrene-isoprene-styrene block copolymers, poly-4-methylpentene, polyisobutylene, polyethylene terephthalate, and cellulose derivatives, and mixtures thereof.

To modify the surface properties of such base polymers, small amounts of the additive need to be utilized. Preferably, about l to 5 parts by weight of the additive is used with 99 to 95 parts by weight of the base polymer. More generally, from .000l to 0.l parts by weight of additive is used with from 0.9999 to 0.9 parts by weight of base polymer.

Methods by which the additives according to the present invention may be mixed with the base polymer include, but are not limited to, melt blending, extrusion blending, blending in solution, particularly when the base polymer is soluble in polar solvents, and by other methods known to those skilled in the art of forming polymer blends.

As discussed above, it is believed that when additives are blended with other polymeric materials and annealed in a low energy environment like air, the low surface energy surface-active component causes the additive to migrate to the surface of the material. It is thus believed that this results in an interface with air dominated by the low surface energy, surface-active component(s), causing the instantaneous contact angles of nonpolar solvents on the surfaces to be relatively high relative to the unmodified base polymer. By contact angle is meant the angle which is measured according to the conventional test between the edge of the drop of a liquid and the surface of the solid material in contact therewith. However, the non-surface-active component is also relatively close to the surface of the material because it is chemically linked (tethered) to surface-active component. Consequently, if the non-surface-active component is polar, the contact angle of polar and nonpolar liquids on the base polymer surface may quickly become relatively low. This is believed possible because of the amphipathic nature imparted to the surface by the additive. Upon contact by a liquid the (modified) surface is capable of reorientation to minimize interfacial energy/contact angle. Thus, certain additives according to the present invention offer the advantages of producing surfaces which are wetted by both polar and nonpolar liquids. An example of a use for products having such properties would be a non-polar substrate (such as polyethylene) paintable by water-based paints.

The additives according to the present invention are also advantageous, for example, in synthetic fibers which are used to form textiles, carpets, outdoor equipment, and the like. Thus, for example, if the additives are added to synthetic polymers to be formed into fibers just prior to the stage at which the fibers are spun, the lubricity required for the spinning and processing of the fibers may be enhanced. For example, since the preferred surface siloxane block is immiscible with many organic materials, the surface of the subsequently formed fiber will be protected from staining from organic materials. Also, being wettable by water, materials made from such fibers would be launderable. Furthermore, when burned, polysiloxanes form a char which is believed may act as a protective flame retardant surface for the fiber. The preferred non-surface-active poly(oxazoline) block may also impart antistatic properties to the fibers, useful, for example, in the formation of carpets.

In addition to the properties described above, the additives according to the present invention are also useful as film-forming surfactants, polishes, biomaterials, adhesives, mold and adhesive release agents, lubricating agents or in other instances wherein changes in surface properties may be desired.

As an example of a particular use of the present invention, in the field of personal care (such as products for hair care) the characteristics of glossiness and smoothness are important, however, many additives which impart these characteristics are not soluble or homogeneously dispersible in alcohol/water solutions which are used in personal care products. It would thus be desirable to obtain an additive which is miscible with or dispersible in a variety of polymer substrates and aqueous solvents and which imparts the characteristics of glossiness and smoothness after the water dispersion or solution is dried. For this particular purpose some polysiloxane oligomers and polysiloxane/poly(alkyleneoxide) surfactants are known to impart glossiness and smoothness to base polymers, but polysiloxanes in general and polysiloxane/poly-(alkyleneoxide) surfactants are not sufficiently miscible with organic base polymers to remain homogeneously dispersed when the alcohol/water solvent is volatilized.

It would also be desirable to obtain an additive in personal care products which at least disperse in water-based emulsions. Polymers cast from emulsions sometimes contain polysiloxane additives to provide surface lubricity, however siloxanes in general and many siloxane copolymers do not disperse in water-based systems. For example, polysiloxane/polyol surfactants which are sometimes used in polyurethane emulsions to form magnetic tape binders, do not remain dispersed in films after the water is evaporated.

The characteristics of surface smoothness, lubricity, stain resistance, flame retardancy, antistatic surface properties, and other surface properties, are desirable in many applications whereby the surface properties are substantially provided by an additive, while the bulk properties are provided by a base polymer or substrate. As an example, in the formation of fibers which may be woven into textile materials such as carpets, outdoor recreational equipment, and the like, it would be desirable to utilize an additive to the fibers used in forming these textiles to enhance the lubricity for spinning the fibers in subsequent processing. It would also be desirable to utilize an additive which would protect the surface of the fiber from staining, and to provide flame retardancy, antistatic properties, and the like.

It would further be desirable to provide an additive which imparts surface properties to a substrate (base polymer) such that the surface is wettable by both polar and nonpolar liquids. Such an additive might allow nonpolar substrates such as polyethylene and polypropylene to be wettable by polar liquids. Thus, for example, using such an additive, polypropylene fibers might be made water-wettable, thus providing improved comfort to fabrics. Another example might be to provide polyethylene and polypropylene parts which would be paintable by water-based paints.

In a preferred embodiment, a method for providing surface glossiness, smoothness and lubricity is provided using novel water miscible additives which are polysiloxane-poly(oxazoline) block copolymers comprising blocks of the formula

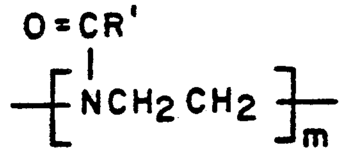

and blocks of the formula

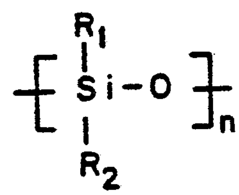

wherein $R_1$ and $R_2$ are alkyl of 1 to 4 carbon atoms, fluoroalkyl of 1 to 4 carbon atoms or aryl of 6 to 10 carbon atoms; R′ is hydrogen, alkyl of 1-4 carbon atoms or aryl of 6 to 10 carbon atoms, and n and m are independently 1 to about 200.

A preferred class of block copolymers includes those of the formula A-B-A
wherein A is

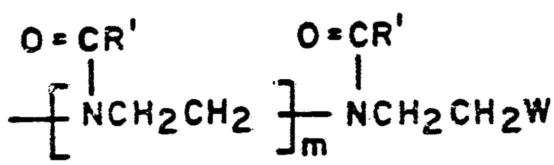

B is

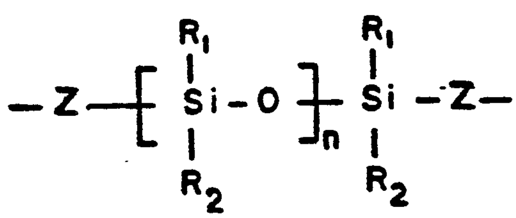

wherein $R_1$ and $R_2$ are alkyl of 1 to 4 carbon atoms, fluoroalkyl of 1 to 4 carbon atoms or aryl of 6 to 10 carbon atoms; n is an integer from 1 to about 200; m is an integer from 1 to about 200; R′ is hydrogen, alkyl of 1 to 4 carbon atoms, or aryl of 6 to 10 carbon atoms; Z is linear or branched alkyl, or aralkyl; and W is halo, aryl of 6 to 10 carbon atoms or alkyl of 1 to 10 carbon atoms.

The following examples are presented to help in the better understanding of the present invention and for purposes of illustration. The examples, however, are not to be construed as limiting the invention to the precise form disclosed or to limit the scope of the invention.

EXAMPLE I

A particularly preferred class of additives comprise novel block copolymers of the formula A-B-A as defined hereinabove. The groups $R_1$ and $R_2$ independently may be an alkyl group of one to four carbon atoms, fluoroalkyl of I to 4 carbon atoms, including linear or branched groups, aryl of 6 to I0 carbon atoms, or combinations thereof. The preferred group is methyl. Aryl groups include, but are not limited to, phenyl, naphthyl, or benzyl. The length of the polysiloxane blocks, as determined by the integer n, is preferably in the range of 5 to I50. The length of the polyoxazoline blocks, as determined by the integer m, is preferably in the range of 5 to 200. The length of the respective blocks (i.e., the values of m and n) may be controlled by the amounts of reactants used, described hereinbelow. The group R' may be hydrogen, linear or branched alkyl of I to 4 carbon atoms or aryl of 6 to I0 carbon atoms, including phenyl, naphthyl, benzyl. Preferably R' is hydrogen or alkyl, with ethyl being the most preferred.

The group W is preferably chloro. Upon polymerization of the oxazoline, initially W will be halo, preferably chloro, but these terminal halo groups may be substituted by conventional reaction to aryl and/or alkyl groups or to any other substituent formable by reaction with a halo group.

The group Z is alkyl, aryl, or aralkyl, preferably of 2 to about 50 carbon atoms. The two Z groups may be the same or different from one another.

The polysiloxane polymers useful as intermediates having a formula as described hereinabove are preferably those wherein $R_1$ $R_2$, Z, and n are the preferred groups having integers defined above in connection with the preferred block copolymers. The group X is preferably chloro. The chloro group may be substituted by conventional reactions to aryl and/or alkyl groups, or to any other substituent formable by reaction with a chloro group.

The polysiloxane polymer intermediates according to the present invention may be prepared by the following steps. Dialkylchlorosilane may be reacted with vinylbenzyl chloride to produce a hydrosilated addition product. Hydrolysis of this addition product forms a benzyl chloride-terminated disiloxane. Reaction of the disiloxane with cyclic siloxanes and a catalytic amount of a strong acid, such as trifluoromethanesulfonic acid, forms the polysiloxane oligomeric intermediates according to the present invention (wherein X is chloro). The molecular weight of the polysiloxane polymers may be controlled by the amounts of the respective reactants utilized. The benzyl halide terminal groups of these polysiloxane intermediates serve as the initiator species, in the presence of a catalytic amount of sodium iodide for initiation of the polymerization of the oxazoline monomer.

To form the polysiloxane-poly(oxazoline) block copolymers according to the present invention, the polysiloxane polymer intermediates are reacted in bulk or in a solvent with, for example, 2-ethyloxazoline to form polysiloxanepoly(oxazoline) block copolymers of the formula A-B-A described hereinabove, wherein R' is ethyl. These block copolymers are physically characterized as glassy, tack-free, film-forming, transparent polymers at room temperature. The block copolymers are soluble in polar solvents such as a mixture of 20% by volume methanol/80% water.

The preferred block copolymers are particularly useful in personal care products since they are miscible with polar base polymers used in these types of products and soluble in alcohol/water solutions. Thus, upon volatilization of these solvents, the block copolymers remain homogeneously dispersed within the base polymer film to retain uniform glossiness and smoothness of the product.

EXAMPLE 2

PREPARATION OF $Cl\text{-}Si(R)_2CH_2CH_2\phi CH_2Cl$

A chloroplatinic acid catalyst solution is prepared by charging a vial with 0.338 g of hydrogen hexachloroplatinate hydrate and dissolving it in I ml dry n-propanol and 5 ml dry diglyme.

A 500 ml round bottomed flask is charged with I46.5 g freshly distilled vinylbenzyl chloride and 250 ul of the above-described catalyst solution. The reaction mixture is stirred under nitrogen for I hour at 30 ± I°C. The solution turns a deep orange color during this stage. Subsequently, 90.6 g distilled dimethylchlorosilane is added in four additions in approximately the following amounts: 24 g, 24 g, 24 g, I8.6 g. Following each addition, the 30°C temperature is maintained until the disappearance of the Si-H absorbance in the infrared spectrum ( 2I32 $c_m$-I). At a pot temperature of II0-II2°C, the product is vacuum distilled at about 0.I mm pressure (b.p.94° C/O.Imm).

EXAMPLE 3

To l.l8 g sodium carbonate dissolved in 20 ml distilled water is added 20 g toluene and 5 g of the product of Example 2. The mixture is interfacially reacted under nitrogen at 65°C for l/2 hour. The phases are separated and the toluene/water azeotrope is distilled from the product phase at a pot temperature of l00-l25°C over a period of l l/2 hours. Residual toluene is vacuum stripped from the product at 60°C. The product is the benzyl chloride functional disiloxane to be used as the endblocker in the redistribution reaction of the benzyl chloride terminated polydimethysiloxane oligomers.

EXAMPLE 4

To form an oligomer of about 2500 molecular weight, a flask is charged with l8.65 g of the benzyl chloride-terminated disiloxane (the product of Example 3), and 87.84 g octamethylcyclotetrasiloxane ($D_4$) and heated to 60°C. Forty-six ul trifluoromethanesulfonic acid is added and the reaction temperature is maintained under nitrogen with agitation for 30 hours.

The mixture is washed three times with water and extracted with diethyl ether to remove residual catalyst. The diethyl ether is evaporated under vacuum and residual $D_4$, $D_5$ and higher cyclics is vacuum distilled at approximately l00°C/20u. The amount of cyclics remaining in the equilibrate at the end of the reaction is about 5-l0% of the total reaction mixture. A clear oligomer results, having a unimodal, approximately gaussian molecular weight distribution and a number average molecular weight of approximately 2300 g/mole.

EXAMPLE 5

Preparation of a Polysiloxane/Poly(2-ethyl)oxazoline Block Copolymer

A resin kettle equipped with a nitrogen inlet and an agitator suitable for high viscosity polymers is charged with 85.6 g of the polysiloxane oligomer from Example 4, l79.8 distilled 2-ethyloxazoline, and 0.255 g sodium iodide. The mixture is reacted at l30°C for l7 hours. A viscous, clear, yellow block copolymer results.

EXAMPLE 6

Films of polyvinylchloride with and without various amount of the surface-modifying block copolymer additives were solution cast from THF and the water contact angle was measured after being present on the films for l minute and 3 minutes. The data is presented in Table l.

TABLE 1

| Sample | Contact Angle ($H_2O$) 1 min. | 3 min. |
|---|---|---|
| Polyvinylchloride | 83° | 77° |
| Polyvinylchloride + 3% of a polycaprolactone block copolymer | 95° | 93° |
| Polyvinylchloride + 3% of a polysiloxane/poly(2-ethyl)oxazoline block copolymer | 20° | 15° |
| Polyvinylchloride +3% poly(2-ethyl)oxazoline | 64° | 60° |

For the same set of samples advancing water contact angles were measured I minute after being placed on the film, then receding contact angles were measure I I/2 minutes after being placed on the film. The data is shown in Table 2.

TABLE 2

| Sample | Advancing Angle (1 min.) | Receding Angle (1½ min.) |
|---|---|---|
| Polyvinylchloride | 85° | 83° |
| Polyvinylchloride + 3% polysiloxane/polycaprolactone block copolymer | 97° | 93° |
| Polyvinylchloride + 3% polysiloxane/poly(ethyl) oxazoline block copolymer | 17° | 7° |
| Polyvinylchloride + 3% polyethyloxazoline | 65° | 44° |

By comparison, the contact angles of nonpolar liquids on the polymers in Table 2 remain low. These data show that the polyvinylchloride with the polysiloxane-polyoxazoline block copolymer additive possesses a surface rapidly wettable by both polar and non-polar liquids. Despite the presence of siloxane in the surface region, the PVC with polysiloxane-polyoxazoline block copolymer additive is more wettable than the PVC with an equal amount of polyoxazoline homopolymer.

The foregoing description of the preferred embodiments of the present invention is presented for the purposes of illustration and description. It is not intended to be exhaustive or to limit the invention to the precise examples disclosed and obviously many modifications and variations are possible in light of the above teachings. The particular embodiments described above were selected and described in order to best explain the principles of the invention and their practical application to thereby enable others skilled in the art to best utilize the invention in various embodiments and with various modifications as are suited to the particular use contemplated.

## Claims

l. A block polymer additive modified for imparting a predetermined surface-property to a base polymer, the additive comprising a surface-active component which is surface-active in the base polymer, the surface-active component comprising a block polymer chain of molecular weight greater than 400, and a functional component characterized by the predetermined surface property and linked through covalent bonds to the surface-active component, the functional component being non-surface-active in the base polymer, wherein the linkage between the functional component and the surface-active component is of a sufficient length to maintain the functional component sufficiently close to the surface-active component to impart the surface property to the base polymer when the additive is in the base polymer;

the additive being substantially nonexudable from the base polymer, and the additive further being thermoplastic or substantially soluble in an organic solvent and substantially insoluble in aqueous solvents.

2. A block polymer additive according to claim l which is (a) chemically linkable to the base polymer or (b) has a sufficiently high molecular weight and a functional component having sufficient intermolecular interaction with the base polymer to render the additive substantially nonexudable from the base polymer.

3. A block polymer additive according to claim l or claim 2 (b) comprising a high cohesive energy density block characterized by a cohesive energy density, CED, greater than the CED of the base polymer, and by a DP of less than l0 or a molecular weight of less than l0000.

4. A block polymer additive according to any one of the preceding claims comprising two or more different functional components, each functional component imparting a different surface property to the base polymer.

5. A block polymer additive according to any one of the preceding claims wherein the surface-active block polymer chain comprises a polydialkylsiloxane.

6. A block polymer additive according to any one of the preceding claims wherein the surface-active property imparted to the base polymer by the or each functional component is selected from oxidation resistance, radiation resistance, chemical resistance, hydrolytic stability, thermal stability, change in coefficient of friction, reduction of drag, increase of etchability by plasmas, etch resistance by plasmas, stain repellency, lauderability, mold releasing properties, reduction in blocking, wettability, anticoagulant activity, antimicrobial activity, reduced mineralization, static charge dissipation, reduced overgrowth by marine organisms, reduced surface chemical reactivity, increased surface chemical activity, selective adsorption or absorption, selective affinity, reduced toxicity, increased compatibility with biological species, increased growth or viability of cell cultures, increased adhesion, increased bond strength, control of refractive index, control of color intensity, control of dyeability, control of gloss, control of water absorption, change of inbarrier properties, change in permeability, change in tear strength, change in crack propagation, change in tensile properties, change in flammability, change in explosive characteristics, change in tactile properties and "hand", change in rheology, increased film forming ability, control of tack, imparting indicator properties, imparting photosensitivity, imparting magnetic receptivitiy, imparting electrical conductivity and change in dielectric strength.

7. A block polymer additive according to claim 6 wherein said property comprises the reduction of mineralization by calcium in the base polymer.

8. A block polymer additive according to any one of the preceding claims, wherein the additive is a solid at 25°C.

9. A block polymer additive according to any one of the preceding claims wherein the additive comprises block, graft or segmented copolymer configurations.

l0. A polymer composition comprising a base polymer and a block polymer additive according to any one of the preceding claims.

ll. A composition according to claim l0 which is a physical mixture of the additive dispersed in the base polymer, or wherein the additive is chemically bonded to the base polymer.

I2. A method of imparting a surface property to a base polymer, comprising blending the base polymer with an additive according to any one of claims I to I0 and, if necessary, elevating the temperature of the blend to achieve chemical bonding between the base polymer and the additive, a composition having the surface property imparted thereto being thereby obtained.

I3. An article, especially a biomedical device or prosthesis, comprising a polymer composition according to claim II or claim I2 or a composition prepared by a method according to claim I3.

Claims for the following Contracting States : AT; ES

I. A method of making a block polymer additive modified for imparting a predetermined surface property to a base polymer, comprising

(A)

(I) preforming a surface active component which is surface active in said base polymer and comprises a block polymer chain of a molecular weight greater than 400,

(II) preforming a functional component characterized by said predetermined surface property, said functional component being non-surface active in said base polymer,

(III) bonding the surface active component and the functional component directly or indirectly to each other to form the additive, the linkage between the two components being of a sufficient length to maintain the functional component sufficiently close to the surface active component to impart the surface property to the base polymer when the additive is in the base polymer, the additive being substantially nonexudable from the base polymer and further being thermoplastic or substantially soluble in an organic solvent and substantially insoluble in aqueous solvents; or

(B) synthesising a surface active component which is surface active in the base polymer and comprises a block polymer chain of a molecular weight greater than 400, synthesising a functional component <u>in situ</u> directly or indirectly on the surface active component to form the additive, the functional component being characterized by the predetermined surface property and being non-surface active in the base polymer, the linkage between the two components being of a sufficient length to maintain the functional component sufficiently close to the surface active component to impart the surface property to the base polymer when the additive is in the base polymer, and the additive being substantially nonexudable from the base polymer and further being thermoplastic or substantially soluble in an organic solvent and substantially insoluble in aqueous solvents, or

(C) synthesising a functional component which is characterized by the predetermined surface property and is non-surface active in the base polymer, synthesising a surface active component <u>in situ</u> directly or indirectly on the functional component to form the additive, the surface active component being surface active in the base polymer and comprising a block polymer chain of a molecular weight of greater than 400, the linkage between the two components being of a sufficient length to maintain the functional component sufficiently close to the surface active component to impart the surface property to the base polymer when the additive is in the base polymer, and the additive being substantially nonexudable from the base polymer and further being thermoplastic or substantially soluble in an organic solvent and substantially insoluble in aqueous solvents.

2. A method according to claim I, wherein (a) the additive has a sufficiently high molecular weight and the functional component sufficient intermolecular interaction with the base polymer to render the additive substantially nonexudable from the base polymer, or (b) the additive is chemically linkable to the base polymer.

3. A method according to claim 2 (a) which further comprises incorporating in the additive a high cohesive energy density block characterized by a high cohesive energy density, CED, greater than the CED of the base polymer and by a DP of less than I0 or a molecular weight of less than I0,000.

4. A method according to any one of the preceding claims wherein two or more different functional components, each of which imparts a different surface property to the base polymer, are incorporated in the block polymer additive.

5. A method according to any one of the preceding claims wherein the surface active block polymer chain comprises a polydialkysiloxane and/or the surface active property imparted to the base polymer by the or each functional component is selected from oxidation resistance, radiation resistance, chemical resistance, hydrolytic stability, thermal stability, change in coefficient of friction, reduction of drag, increase of etchability by plasmas, etch resistance by plasmas, stain repellency, lauderability, mold releasing properties, reduction in blocking, wettability, anticoagulant activity, antimicrobial activity, reduced mineralization, static charge dissipation, reduced overgrowth by marine organisms, reduced surface chemical reactivity, increased surface chemical actiovity, selective adsorption or absorption, selective affinity, reduced

toxicity, increased compatibility with biological species, increased growth or viability of cell cultures, increased adhesion, increased bond strength, control of refractive index, control of color intensity, control of dyeability, control of gloss, control of water absorption, change of inbarrier properties, change in permeability, change in tear strength, change in crack- propagation, change in tensile properties, change in flammability, change in explosive characteristics, change in tactile properties and "hand", change in rheology, increased film forming ability, control of tack, imparting indicator properties, imparting photosensitivity, imparting magnetic receptivitiy, imparting electrical conductivity and change in dielectric strength.

6. A method according to claim 5 wherein the property imparted to the base polymer comprises the reduction of mineralisation by calcium in the base polymer.

7. A method according to any one of the preceding claims wherein the additive is a solid at 25°C.

8. A method according to any one of the preceding claims, wherein the additive is formed in block, graft or segmented copolymer configurations.

9. A method of forming a polymer composition comprising:

(A) blending a base polymer and a block polymer additive modified for imparting a predetermined surface-property to a base polymer, the additive comprising a surface-active component which is surface active in the base polymer, the surface-active component comprising a block polymer chain of molecular weight greater than 400, and a functional component characterized by the predetermined surface property and linked through covalent bonds to the surface-active component, the functional component being non-surface-active in the base polymer, wherein the linkage between the functional component and the surface-active component is of a sufficient length to maintain the functional component sufficiently close to the surface-active component to impart the surface property to the base polymer when the additive is in the base polymer; the additive being substantially nonexudable from the base polymer, and the additive further being thermoplastic or substantially soluble in an organic solvent and substantially insoluble in aqueous solvents and, if necessary, the method further comprising elevating the temperature of the blend to achieve chemical bonding between the base polymer and the additive, a composition having a surface property imparted thereto by the additive being thereby obtained; or

(B) carrying out a method according to any one of the preceding claims and blending the resultant additive with a base polymer and, if necessary, elevating the temperature of the blend to achieve chemical bonding between the base polymer and the additive, a composition having a surface property imparted thereto by the additive being thereby obtained.

10. An article comprising a polymer composition prepared by a method according to claim 9 or a composition comprising a base polymer and a block polymer additve as defined in claim 9 (A).

11. A biomedical device, a prosthesis, synthetic fibers, textile material, magnetic tape or an article coated with water-based paint comprising a composition comprising a base polymer and block polymer additive as defined in claim 9(A).

12. The use of a block polymer additive as defined in claim 9(A) to impart a surface property to a base polymer.